# EUROPÄISCHE PATENTANMELDUNG

(11) **EP 0 808 834 A1**
(43) Veröffentlichungstag der Anmeldung: **26.11.1997**
(21) Anmeldenummer: 97107776.3
(22) Anmeldetag: 13.05.1997
(51) Int. Cl.: C07D 209/86

(54) **Verfahren zur Herstellung von Carbazol**

(30) Priorität: 24.05.1996 DE 19620990
(71) Anmelder: BAYER AG, 51368 Leverkusen (DE)
(72) Erfinder: Schnatterer, Albert, Dr., 51373 Leverkusen (DE); Fiege, Helmut, Dr., 51373 Leverkusen (DE); Jentsch, Joerg-Dietrich, Dr., 45468 Mülheim (DE); Zirngiebl, Eberhard, Dr., 51061 Köln (DE)

(57) **Zusammenfassung**

Diphenylamin läßt sich an Edelmetallkatalysatoren in flüssiger Phase bei Temperaturen von 200 bis 340°C dehydrierend cyclisieren. Die Reaktion verläuft mit überraschend hoher Selektivität.

## Beschreibung

Die Erfindung betrifft die Herstellung von Carbazol durch dehydrierende Cyclisierung von Diphenylamin in flüssiger Phase.

Carbazol ist ein wichtiges Zwischenprodukt zur Herstellung von Farbstoffen, Pigmenten, Pestiziden und Polymeren.

Carbazol ist im Hochtemperatur-Steinkohlenteer im Durchschnitt zu 1,5 Gew.-% enthalten. Bei der Teerdestillation fällt es angereichert in der Anthracenöl-Fraktion an und kann aus den Mutterlaugen nach der Anthracenkristallisation isoliert werden. Bisher wurde der technische Bedarf an Carbazol überwiegend aus dieser Quelle gedeckt (Ullmann's Encyclopedia of Industrial Chemistry, 5. Edition, Volume A 5, 1986, S. 59). Ein solches Verfahren ist jedoch mit einem enormen Zwangsanfall von Begleitstoffen gekoppelt; Anthracenöl aus der Teerdestillation enthält außer Anthracen und Carbazol noch angereicherte Mengen Phenanthren, Pyren und Fluoranthen und ist deshalb eine unbefriedigende Basis für eine langfristige technische Versorgung.

Daher hat es bereits in der Vergangenheit nicht an Versuchen gefehlt, Carbazol durch gezielte Synthesen herzustellen, beispielsweise durch Dehydrierung und Cyclisierung von Diphenylamin, o-Aminodiphenyl oder N-Cyclohexylidenanilin (Ullmann's Encyclopedia of Industrial Chemistry, 5. Edition, Volume A 5, S. 59).

Die Synthese von Carbazol ausgehend von Diphenylamin ist wegen der preisgünstigen Verfügbarkeit des Diphenylamins besonders interessant. Die Bildung von Carbazol beim Durchleiten von Diphenylamin durch ein glühendes Porzellanrohr wurde bereits 1872 beschrieben (Graebe, Ber. dt. Chem. Ges. 5 (1872), 377). In der Zwischenzeit sind verschiedene Katalysatoren und Reaktionsbedingungen für die Gasphasendehydrierung und -cyclisierung des Diphenylamins publiziert worden, beispielsweise Platin auf Kohle (Zelinsky; Titz; Graverdoskaja; Ber. dt. Chem. Ges. 59 (1926), 2592), Zinkoxid auf Aluminiumoxid, Molybdänsulfid auf Aluminiumoxid und Wolframsulfid (DE 937 590), Platin (US 2 921 942).

Gasphasenreaktionen haben jedoch den Nachteil, daß sie eine spezielle Ausrüstung mit einem für den jeweiligen Reaktionstyp maßgeschneiderten Reaktor und Katalysator erfordern. Somit setzen Prozesse in der Gasphase eine erhebliche finanzielle Investition voraus.

Dies gilt im besonderen auch für den Prozeß der Gasphasendehydrierung und -cyclisierung von Diphenylamin zu Carbazol. Da das Produkt hochschmelzend ist (Fp. 245°C), müssen u.a. kristallisationsverhindernde Vorkehrungen getroffen werden, beispielsweise durch entsprechend hohe Temperierung von Reaktorteilen oder durch Einbringung eines Lösungsmittels.

Überraschenderweise wurde nun gefunden, daß bei der dehydrierenden Cyclisierung von Diphenylamin zu Carbazol auch in flüssiger Phase gearbeitet werden kann. Dadurch läßt sich die Reaktion prinzipiell in einfachen Rührreaktoren durchführen.

Gegenstand der Erfindung ist deshalb ein Verfahren zur Herstellung von Carbazol aus Diphenylamin, dadurch gekennzeichnet, daß Diphenylamin in flüssiger Phase mit einem Edelmetallkatalysator bei 200 bis 340°C in Kontakt gebracht wird.

Der Edelmetallkatalysator wird vorzugsweise aus Platin, Palladium und deren Verbindungen gewählt. Er kann in Form der Metalle beispielsweise fein verteilt als Platin-Schwarz oder Palladium-Schwarz oder in Form von Verbindungen dieser Metalle beispielsweise als Metallsalz oder Metallkomplex eingesetzt werden. Solche Einsatzformen des Edelmetalls können auch auf Trägern aufgebracht sein.

Bevorzugte Edelmetallverbindungen umfassen beispielsweise die Platinverbindungen PtO₂, H₂PtCl₆, PtCl₂, PtCl₄, PtBr₂, Pt(NH₃)₂(NO₂)₂, PtJ₂, Pt(NH₃)₄Cl₂, Pt(H₂NCH₂CH₂NH₂)₂Cl₂ und die Palladiumverbindungen PdO, PdSO₄, PdBr₂, PdCl₂, Pd(CH₃CO₂)₂, Pd(NH₃)₄(NO₃)₂, Palladium(II)acetylacetonat und Palladium-(II)-trifluoracetat.

Als Katalysatorträger eignen sich alle technisch üblichen Katalysatorträger, z.B. solche auf der Basis von Kohle, Elementoxiden, Elementcarbiden oder Elementsalzen in verschiedenen Anwendungsformen. Beispiele für Elementoxid-Katalysatorträger sind Siliciumdioxid (natürliche oder synthetische Kieselsäuren, Quarz), Aluminiumoxid (α,γ-Al₂O₃), Tonerden, natürliche und synthetische Alumosilikate (Zeolithe), Titandioxid (Rutil, Anatas), Zirkondioxid oder Zinkoxid. Bevorzugte Elementcarbide und -salze umfassen Siliciumcarbid, Aluminiumphosphat, Bariumsulfat, Calciumcarbonat u.a. Sie können sowohl im Sinne chemisch einheitlicher Reinsubstanzen als auch im Gemisch eingesetzt werden. Erfindungsgemäß als Katalysatorträger eignen sich sowohl stückige als auch pulverförmige Materialien.

Das Aufbringen des Edelmetalls auf den Katalysatorträger kann nach grundsätzlich bekannten Methoden erfolgen. So können eine oder mehrere der Edelmetallverbindungen beispielsweise durch Tränken, Absorption, Tauchen, Sprühen, Imprägnieren und Ionenaustausch auf den Katalysatorträger gebracht werden. Es ist weiterhin möglich, die Metalle durch Fällung mit einer Base auf dem Träger zu fixieren. Als Base kommen z.B. (Erd-)Alkalimetallhydroxide wie Calcium-, Magnesium-, Natrium-, Lithium- und Kaliumhydroxid, (Erd-)Alkalimetallhydrogencarbonate wie Calcium-, Magnesium-, Natrium-, Lithium- und Kaliumhydrogencarbonate, (Erd-)Alkalicarbonate wie Calcium-, Magnesium-, Natrium-, Lithium- und Kaliumcarbonat sowie Alkalimetallsalze schwacher Säuren (z.B. Essigsäure) wie Natrium- und Kaliumacetat in Frage.

Die Edelmetallkatalysatoren können entweder in Form der Metallverbindungen, gegebenenfalls auf einem der genannten Träger, direkt eingesetzt werden oder vor der Verwendung reduziert werden. Als Reduktionsmittel kommen beispielsweise Hydrazin, Formaldehyd, Natriumformiat, Natriumborhydrid bei Temperaturen von 0 bis 200°C oder gasförmiger Wasserstoff bei Temperaturen von 0 bis 500°C, vorzugsweise 20 bis 300°C, in Frage.

Der Einsatz der Katalysatoren auf Trägern wird bevorzugt; besonders bevorzugt ist der Einsatz von Platin und Platinverbindungen auf Trägern.

Die Beladung des Trägers mit Edelmetall(verbindung) beträgt vorzugsweise 0,1 bis 15, insbesonders 0,5 bis 10 Gew.-%, bezogen auf die Summe von Träger und Edelmetall(verbindung), berechnet als Metall.

Der Edelmetallkatalysator wird in Mengen von 0,001 bis 2, vorzugsweise 0,01 bis 1 Gew.-%, berechnet als Metall und bezogen auf Diphenylamin, eingesetzt.

Das erfindungsgemäße Verfahren wird vorzugsweise bei einer Temperatur von 220 bis 310, insbesondere 240 bis 300°C, ausgeführt.

Das erfindungsgemäße Verfahren läßt sich in technischen Anlagen auf vielfältige Weise durchführen. In der einfachsten Ausführungsform wird das Diphenylamin (Fp. 52°C, Sdp. 302°C) mit dem Katalysator auf Reaktionstemperatur gebracht, beispielsweise unter Rühren in einem zur Handhabung von Flüssigkeiten geeigneten Reaktor. Ein Verdünnungsmittel ist nicht verfahrensnotwendig, kann jedoch die Handhabung des Reaktionsgemisches vereinfachen.

Als Verdünnungsmittel kommen prinzipiell alle hochsiedenden organischen Verbindungen und Gemische von Verbindungen in Frage, die über einen hinreichenden Temperaturbereich flüssig sind und unter den Reaktionsbedingungen ausreichend stabil sind. Als preisgünstige Verdünnungsmittel bieten sich beispielsweise isomere Terphenyle, isomere Diisopropylnaphthaline, isomere Ditolylether, Polyethylenglykol, Diphenyl und Diphenylether an.

Der Druck ist im erfindungsgemäßen Verfahren keiner spezifischen Einschränkung unterworfen. Da es sich um eine Reaktion unter Freisetzung von Wasserstoff handelt, mag es zweckmäßig sein, das Druckniveau niedrig zu halten. Gegebenenfalls kann auch eine leichte Druckabsenkung hilfreich sein.

Bevorzugt wird jedoch im Bereich des Atmosphärendrucks gearbeitet.

Der freigesetzte Wasserstoff kann als Abgas ausgeschleust werden, kann aber auch im Sinne einer Wasserstoff-Transferreaktion auf geeignete Akzeptoren übertragen werden. Eine beschleunigte Abfuhr des Wasserstoffes kann auch dadurch herbeigeführt werden, daß ein Inertgasstrom, bestehend beispielsweise aus Stickstoff oder Kohlendioxid, durch das Reaktionsgemisch geleitet wird.

Überraschenderweise gelingt die Dehydrocyclisierung von Diphenylamin zu Carbazol nach dem erfindungsgemäßen Verfahren auch in flüssiger Phase mit hoher Umsetzungsgeschwindigkeit. Wegen der vergleichsweise hohen Verweilzeit des Reaktionsgemisches in der Flüssigphase und der damit verbundenen thermischen Belastung war nicht zu erwarten, daß Carbazol in so hoher Selektivität gebildet wird.

### Beispiele

Die Prozentangaben der nachfolgenden Beispiele beziehen sich jeweils auf das Gewicht.

### Beispiel 1

In einem 250 ml Glaskolben mit Thermometer und Destillationsbrücke wurden 100 g Diphenylamin und 10 g 5 % Pt/Kohle (K-0101 von Haereus, 50 % Wasser) vorgelegt. Nach Spülen der Apparatur mit Stickstoff wurde das Gemisch unter Rühren 5 Stunden auf 290°C erhitzt. Dabei destillierten 13 g Leichtsieder ab, hauptsächlich Wasser (aus dem Katalysator), Benzol und etwas Anilin.

Nach dem Abkühlen wurde der Rückstand in 400 ml Aceton aufgenommen, der Katalysator abfiltriert und das Filtrat eingedampft. Als Rückstand blieben 86,6 g kristalliner Feststoff vom Gehalt (GC):
- 10,2 %: Anilin
- 39,4 %: Diphenylamin
- 45,1 %: Carbazol.

Dies entspricht einer Carbazolselektivität von 60,0 % bei einem Diphenylaminumsatz von 65,9 %.

### Beispiel 2

In einem 0,7 l VA-Reaktor, der mit einem wassergekühlten Kondensator ausgerüstet war, wurden 200 g Diphenylamin und 10 g 1 % Pt/γ-Al₂O₃ vorgelegt. Nach Spülen des Reaktors mit Stickstoff wurde das Gemisch drucklos unter Rühren 5 Stunden auf 250°C erhitzt. Es wurden während der Reaktionsphase 2,6 g Leichtsieder abgetrennt. Nach dem Abkühlen wurde das Reaktionsgemisch in 400 ml Aceton aufgenommen, der Katalysator abfiltriert und das Filtrat eingedampft. Als Rückstand blieben 192,8 g kristalliner Feststoff vom Gehalt (GC):
- 0,8 %: Anilin
- 91,4 %: Diphenylamin
- 5,8 %: Carbazol.

Dies entspricht einer Carbazolselektivität von 47,6 % bei einem Diphenylaminumsatz von 11,9 %.

## Patentansprüche

1. Verfahren zur Herstellung von Carbazol aus Diphenylamin, dadurch gekennzeichnet, daß Diphenylamin in flüssiger Phase mit einem Edelmetallkatalysator bei 200 bis 340°C in Kontakt gebracht wird.

2. Verfahren nach Anspruch 1, dadurch gekennzeichnet, daß der Edelmetallkatalysator aus Platin, Palladium und deren Verbindungen gewählt wird.

3. Verfahren nach Anspruch 1, dadurch gekennzeichnet, daß der Edelmetallkatalysator auf einem Katalysatorträger eingesetzt wird.

4. Verfahren nach Anspruch 3, dadurch gekennzeichnet, daß als Edelmetallkatalysator Platin und als Katalysatorträger Kohle, Aluminiumoxid oder Siliciumdioxid eingesetzt werden.

5. Verfahren nach Anspruch 1, dadurch gekennzeichnet, daß im Temperaturbereich 220 bis 310°C gearbeitet wird.
